# EUROPEAN PATENT APPLICATION

(11) **EP 1 961 375 A1**
(43) Date of publication of application: **27.08.2008**
(21) Application number: 08002462.3
(22) Date of filing: 11.02.2008
(51) Int. Cl.: A61B 5/021

(54) **Vibration detecting device**

(30) Priority: 22.02.2007 JP 2007042827
(71) Applicant: Alps Electric Co., Ltd., Tokyo 145-8501 (JP)
(72) Inventor: Suzuki, Testuya, Tokyo 145-8501 (JP)
(74) Representative: Klunker . Schmitt-Nilson . Hirsch

(57) **Abstract**

Disclosed is a vibration detecting device capable of accurately detecting vibration regardless of the position of an object to be measured, without alignment with a part of the human body to be measured.

A case has a sufficient size to be mounted to a part of the human body to be measured. In an embodiment, since it is assumed that a vibration detecting device is worn on a user's wrist, the case has a ring shape with a sufficient size to be worn on the user's wrist. Rubber, which is an elastic material, is provided in the case, and piezoelectric elements are provided inside the rubber. The piezoelectric element is an electric field changing unit that changes an electric field using the vibration of an object to be measured. Electric field sensors that detect the electric field are provided on the outer surface of the case.

## Description

This application claims benefit of the Japanese Patent Application No. 2007-042827 filed on February 22, 2007, the entire content of which is hereby incorporated by reference.

### BACKGROUND

### 1. Field of the Invention

The present invention relates to a vibration detecting device that detects vibration such as pulsation.

### 2. Description of the Related Art

For example, a device that detects vibration, such as pulsation, has been disclosed in JP-A-2000-342547 and JP-A-2001-57965. Such a device measures a pulse wave by emitting light from a light-emitting element to a part of the human body to be measured, such as a finger of an arm, and detecting light passing through or reflected from hemoglobin in the blood flowing through blood vessels.

However, in the optical pulse wave measuring method according to the related art, it is necessary to align the light-emitting element with the blood vessel, accurately position the device, and align the optical axis of the light-emitting element with the optical axis of a light-receiving element. In addition, in the optical pulse wave measuring method, when the blood vessels are deep in the skin, it is difficult to accurately measure the pulse wave.

### SUMMARY

An object of the invention is to provide a vibration detecting device capable of accurately detecting vibration regardless of the position of an object to be measured, without alignment with a part of the human body to be measured.

According to an aspect of the invention, a vibration detecting device includes: an electric field changing unit that changes an electric field using the vibration of an object to be measured; an electric field detecting unit that detects the electric field changed by the electric field changing unit; and a converting unit that converts the variation in the electric field into waveform data.

According to the above-mentioned structure, since a variation in the electric field corresponding to vibration is detected, the alignment or positioning of the optical axes is not needed unlike the optical pulse wave measuring method, and it is possible to accurately detect vibration with a simple mounting structure. In addition, since the variation in the electric field corresponding to vibration is detected, it is possible to accurately measure a pulse wave even when the blood vessel is deep in the skin, for example.

In the vibration detecting device according to the above-mentioned aspect, preferably, the electric field changing unit is a piezoelectric element.

In the vibration detecting device according to the above-mentioned aspect, preferably, the electric field changing unit includes a magnetic material with elasticity and a coil that is disposed at a position that is affected by a magnetic field generated by the magnetic material.

In the vibration detecting device according to the above-mentioned aspect, preferably, the vibration is a pulse wave.

According to the invention, the vibration detecting device includes the electric field changing unit that changes an electric field using the vibration of an object to be measured, the electric field detecting unit that detects the electric field changed by the electric field changing unit, and the converting unit that converts the variation in the electric field into waveform data. Therefore, the vibration detecting device is capable of accurately detecting vibration regardless of the position of an object to be measured, without alignment with a part of the human body to be measured.

### BRIEF DESCRIPTION OF THE DRAWINGS

Fig. 1 is a diagram illustrating a pulse wave sensor, serving as a vibration detecting device according to a first embodiment of the invention;
Fig. 2 is a block diagram schematically illustrating the structure of the pulse wave sensor shown in Fig. 1;
Fig. 3 is a diagram illustrating the waveform of a pulse wave;
Figs. 4A and 4B are diagrams illustrating the patterns of data obtained by differentiating the pulse wave twice;
Fig. 5 is a diagram illustrating a pulse wave sensor, serving as a vibration detecting device according to a second embodiment of the invention; and
Fig. 6 is a block diagram schematically illustrating the structure of the pulse wave sensor shown in Fig. 5.

### DESCRIPTION OF THE EMBODIMENTS

Hereinafter, exemplary embodiments of the invention will be described in detail with reference to the accompanying drawings. In the embodiments, the pulse of the human body is used as vibration.

### (First embodiment)

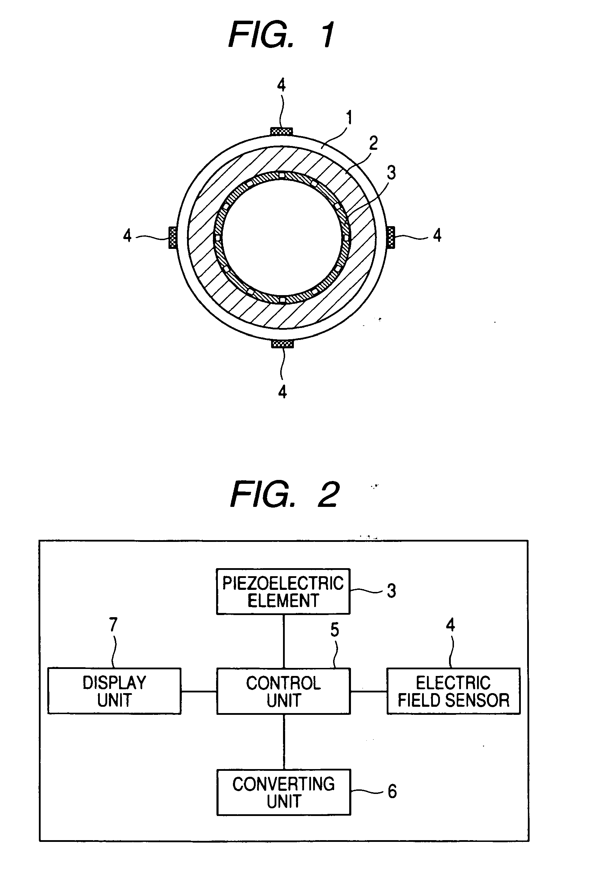

Fig. 1 is a diagram illustrating a pulse wave sensor, serving as a vibration detecting device according to a first embodiment of the invention, and Fig. 2 is a block diagram schematically illustrating the structure of the pulse wave sensor shown in Fig. 1.

The pulse wave sensor shown in Fig. 1 has a case 1. The case 1 has a sufficient size to be mounted to an object to be measured. In this embodiment, since it is assumed that the vibration detecting device is worn on a user's wrist, the case 1 has a ring shape with a sufficient size to be worn on the user's wrist.

Rubber 2, which is an elastic material, is provided in the case 1, and a piezoelectric element 3 is provided inside the rubber 2. The piezoelectric element 3 is an electric field changing unit that changes an electric field using the vibration of an object to be measured. In addition, an electric field sensor 4 that detects the electric field is provided on the outer surface of the case 1. In this embodiment, a plurality of piezoelectric elements 3 are provided inside the rubber 2, and a plurality of electric field sensors 4 are provided on the outer surface of the case 1.

As shown in Fig. 2, the pulse wave sensor having the above-mentioned structure includes a control unit 5 that controls the operation of all the sensors, a converting unit 6 that converts a variation in the electric field into waveform data, particularly, pulse wave data in this embodiment, and a display unit 7 that displays the converted pulse wave data.

When the pulse wave sensor having the above-mentioned structure detects a pulse wave, the pulse wave sensor shown in Fig. 1 is worn on the wrist. In this case, the piezoelectric elements 3 come into contact with the wrist, and the rubber 2 and the piezoelectric elements 3 are deformed due to the pulsation of blood vessels. When the piezoelectric elements 3 are deformed, the electric field around the piezoelectric elements is changed. The variation in the electric field is detected by the electric field sensors 4. The variation in the electric field detected by the electric field sensors 4 is converted into pulse wave data by the converting unit 6. The display unit 7 displays the pulse wave data.

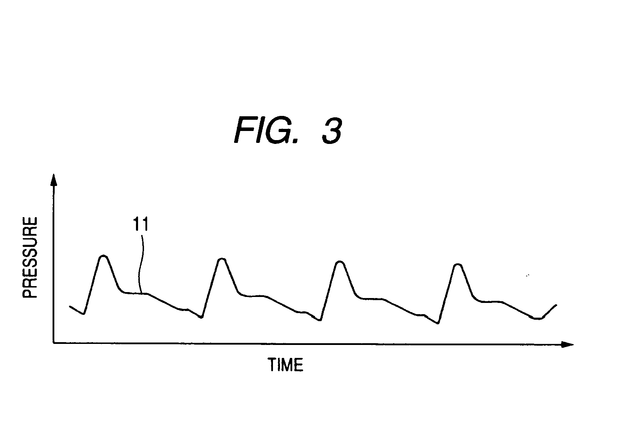

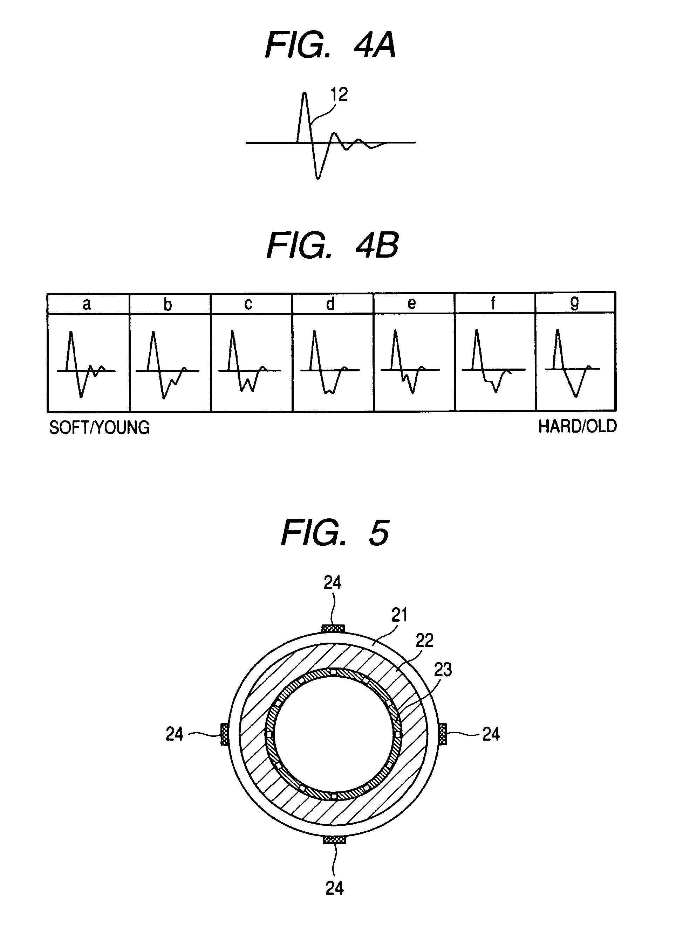

The pulse wave indicates a variation in the pressure of the blood vessel due to blood pumped out from the heart. Therefore, the pulse wave data is represented by a waveform 11 showing the relationship between pressure and time shown in Fig. 3. A velocity pulse wave is obtained by differentiating the pulse wave once, and an acceleration pulse wave is obtained by differentiating the pulse wave twice. As shown in Fig. 4A, the acceleration pulse wave includes several peaks 12. The pattern of the peaks makes it possible to monitor the state of blood vessels. For example, it is possible to check the state of the blood vessels on the basis of pulse wave patterns A to G shown in Fig. 4B. That is, the pattern A indicates a soft and young blood vessel, and the pattern G indicates a hard and old blood vessel.

As described above, the pulse wave sensor according to this embodiment can detect a variation in the electric field corresponding to vibration, such as pulsation. Therefore, the alignment or positioning of optical axes alignment or is not needed unlike an optical pulse wave measuring method, and it is possible to accurately detect vibration, such as pulsation, using a simple mounting structure. In addition, since the pulse wave sensor detects a variation in the electric field corresponding to vibration, such as pulsation, it is possible to accurately detect the pulse of the blood vessel that is deep in the skin.

### (Second embodiment)

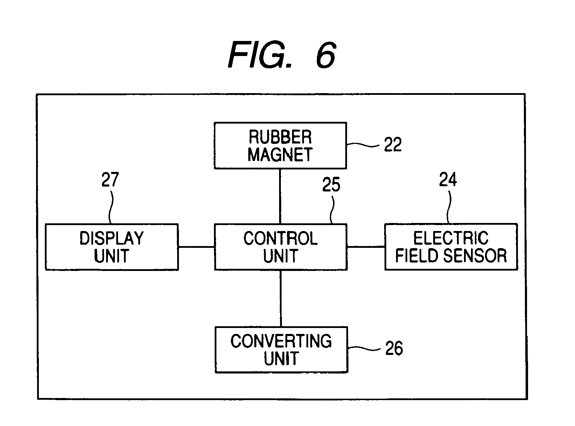

Fig. 5 is a diagram illustrating a pulse wave sensor, serving as a vibration detecting device according to a second embodiment of the invention, and Fig. 6 is a block diagram schematically illustrating the structure of the pulse wave sensor shown in Fig. 5.

The pulse wave sensor shown in Fig. 5 includes a case 21. The case 21 has a sufficient size to be mounted to an object to be measured. In this embodiment, since it is assumed that the vibration detecting device is worn on a user's wrist, the case 21 has a ring shape with a sufficient size to be worn on the user's wrist.

A rubber magnet 22, which is a magnetic material with elasticity, is provided in the case 21, and a coil 23 is provided inside the rubber magnet 22. The rubber magnet 22 and the coil 23 form an electric field changing unit that changes an electric field using the vibration of an object to be measured. In addition, an electric field sensor 24 that detects the electric field is provided on the outer surface of the case 21. In this embodiment, a plurality of electric field sensors 24 are provided on the outer surface of the case 21. The positions of the rubber magnet 22 and the coil 23 are not limited to the above. The coil 23 and the rubber magnet 22 may be provided at any positions that are affected by the magnetic field.

As shown in Fig. 6, the pulse wave sensor having the above-mentioned structure includes a control unit 25 that controls the operation of all the sensors, a converting unit 26 that converts a variation in the electric field into waveform data, particularly, pulse wave data in this embodiment, and a display unit 27 that displays the converted pulse wave data.

When the pulse wave sensor having the above-mentioned structure detects a pulse wave, the pulse wave sensor shown in Fig. 5 is worn on the wrist. In this case, the rubber magnet 22 having the coil 23 comes into contact with the wrist, and the rubber magnet 22 is deformed due to the pulsation of blood vessels. When the rubber magnet 22 is deformed, a magnetic field around the rubber magnet 22 is changed. The variation in the magnetic field causes an induced electromotive force to be generated in the coil 23. The induced electromotive force changes the electronic field. The variation in the electric field is detected by the electric field sensors 24. The variation in the electric field detected by the electric field sensors 24 is converted into pulse wave data by the converting unit 26. The display unit 27 displays the pulse wave data.

As described above, the pulse wave sensor according to this embodiment can detect a variation in the electric field corresponding to vibration, such as pulsation. Therefore, the alignment or positioning of optical axes is not needed unlike an optical pulse wave measuring method, and it is possible to accurately detect vibration, such as pulsation, using a simple mounting structure. In addition, since the pulse wave sensor detects a variation in the electric field corresponding to vibration, such as pulsation, it is possible to accurately detect the pulse of the blood vessel that is deep in the skin.

The invention is not limited to the above-described embodiments, but various modifications and changes of the invention can be made. In the first and second embodiments, the pulse wave device is worn on the wrist, but the invention is not limited thereto. The pulse wave device may be worn on the finger or arm. In this case, the size of the case depends on the size of a part of the human body on which the case is worn. In addition, in the first and second the embodiments, the case is formed in a ring shape, but the invention is not limited thereto. For example, layers (rubber, a piezoelectric element, a rubber magnet, and a coil) may be formed on a case, and the laminate may be fixed in a ring shape according to the thickness of a part of the human body on which the laminate is worn.

Further, in the above-described embodiments, the pulsation of the human body is used as vibration, but the invention is not limited thereto. The invention can be applied to vibrations other than the pulsation. In addition, it will be understood by those skilled in the art that the number of components, materials forming the components, the structure of a processing unit, and a processing sequence may be appropriately changed without departing from the scope and spirit of the invention. Other components may be appropriately changed or modified without departing from the scope of the invention.

## Claims

1. A vibration detecting device comprising:
an electric field changing unit that changes an electric field using the vibration of an object to be measured;
an electric field detecting unit that detects the electric field changed by the electric field changing unit; and
a converting unit that converts the variation in the electric field into waveform data.

2. The vibration detecting device according to claim 1,
wherein the electric field changing unit is a piezoelectric element.

3. The vibration detecting device according to claim 1,
wherein the electric field changing unit includes:
a magnetic material with elasticity; and
a coil that is disposed at a position that is affected by a magnetic field generated by the magnetic material.

4. The vibration detecting device according to any one of claims 1 to 3,
wherein the vibration is a pulse wave.
